# EUROPEAN PATENT APPLICATION

(11) **EP 4 428 827 A1**
(43) Date of publication of application: **11.09.2024**
(21) Application number: 23160440.6
(22) Date of filing: 07.03.2023
(51) Int. Cl.: G06T 19/00, G06T 15/08, G16H 30/00

(54) **APPARATUS FOR GENERATING A VISUALIZATION OF A MEDICAL IMAGE VOLUME**

(71) Applicant: Koninklijke Philips N.V., 5656 AG Eindhoven (NL)
(72) Inventor: HABETS, Hugo Frans, Eindhoven (NL)
(74) Representative: Philips Intellectual Property & Standards

(57) **Abstract**

The invention refers to an apparatus for generating a visualization of a medical image volume, e.g. MRI, CT, PET. A providing unit 111 provides image data defining an image volume of a region of interest. A generation unit 112 generates a surface image based on the image data defining one or more surfaces in the image volume. A generation unit 113 generates a visualization value for a surface point on a surface in the surface image, wherein the visualization value is generated based on at least one value of the image data associated with a location positioned at a distance from a location of the surface point and outside the surface itself. A generation unit 114 generates a visualization of the image volume based on the surface image and the generated visualization value. This allows to increase the density of the visualized information to a user in one image.

## Description

### FIELD OF THE INVENTION

The invention refers to an apparatus, a method and a computer program product for generating a visualization of a medical image volume.

### BACKGROUND OF THE INVENTION

Medical scan data, for example, from MRI, CT or PET, generally refer to data acquired in a 3D volume. Usually, this 3D volume data is visualized by utilizing image slices through the 3D volume representing the data as 2D slice images or by volume rendering a 3D view into a 2D image. In some cases, also a combination, for instance, cutting through a volumetric rendering, can be utilized. However, all of these visualization methods are limited in the information that is provided to a user, for instance, a surgeon. For example, a 2D slice through a volume image can provide a plurality of details of the image data but is missing the spatial context of these details, in particular, the 3D relation of the information to other structures in the 3D volume. The 3D rendering provides a good overview of the spatial relation between different structures in the 3D volume, however is missing details with respect to structures with different densities in the 3D volume. Thus, in order to be provided with all possible information from a respective medical scan, a user often has to toggle between different kinds of visualizations which is a cumbersome and time-consuming task and still provides the possibility of missing some important information if the experience of the user is not high enough. It would therefore be advantageous if a visualization provides a user with the information from medical scans in an intuitive way and reduces the risk of missing important information.

### SUMMARY OF THE INVENTION

It is an object of the present invention to provide an apparatus, a method and a computer program product that visualizes medical 3D data in an intuitive way reducing the risk of missing important information.

In a first aspect, an apparatus is presented for generating a visualization of a medical image volume, wherein the apparatus comprises a) a medical image data providing unit for providing medical image data acquired from a subject in a medical imaging procedure, wherein the medical image data defines a medical image volume of a region of interest of the subject, b) a surface generation unit for generating a surface image based on the medical image data defining one or more surfaces in the medical image volume of the region of interest, c) a surface value generation unit for generating a visualization value for a surface point on a surface in the surface image, wherein the visualization value is generated based on at least one value of the medical image data associated with a location positioned at a distance from a location of the surface point and outside the surface itself, and d) a visualization generation unit for generating a visualization of the medical image volume based on the surface image and the generated visualization value.

Since first a surface is generated in the medical image volume and then a visualization value is generated for a surface point that is associated with the location positioned at a predetermined distance from a location of the surface point and outside the surface itself, a visualization can be provided that provides a user not only with the information on the spatial relation between structures in the medical image but also with the information on possible important features that are not directly shown on the surface. Thus, a user will not miss a feature because it is positioned slightly below or above the utilized surface from the perspective of the user. This allows to increase the density of the visualized information to a user in one image such that the user can grasp the information intuitively and the chances of missing important information in the medical image data is reduced.

The apparatus is generally configured for generating a visualization of a medical image volume. The apparatus can be realized in form of any software and/or hardware that is configured for performing the functions of the apparatus. In particular, the apparatus can be realized in form of one or more computer processors that are configured to performing the functions of the respective units of the apparatus. Moreover, the functions performed by the apparatus can also be performed by more than one computing unit, for instance, in a cloud computing or distributed computing environment.

The medical image data providing unit is configured to provide medical image data. For example, the medical image data providing unit can be configured for accessing medical image data that is stored on a respective storage unit for providing the same. Moreover, the medical image data providing unit can be configured for directly receiving the medical image data from a medical image acquisition unit acquiring the medical image data, for example, via a respective interface, and after receiving the medical image data the medical image providing unit can provide the same. Furthermore, the medical image data providing unit can itself be a medical image acquisition unit or a part of a medical image acquisition unit acquiring the medical image data and providing the same. Generally, the medical image data is acquired from a subject in a medical imaging procedure. The subject can be any living being, for instance, a human or an animal. The medical imaging procedure can be any procedure that allows to acquire medical image data that defines a medical image volume of a region of interest of a subject. For example, the medical imaging procedure can be a magnetic resonance imaging, a computed tomography imaging, a positron emission tomography imaging, a 3D ultrasound imaging, a 3D x-ray imaging, etc. The region of interest of the subject can be any region that is of interest, for instance, can comprise one or more organs, anatomical structures, parts of the body, etc. Preferably, the region of interest is a head of a subject.

The surface generation unit is configured for generating a surface image based on the medical image data defining one or more surfaces in the medical image volume of the region of interest. A surface is defined by defining points in the medical image data that belong to the respective surface and storing the definition as surface image. Generally, a surface image refers to an image that can be visualized by surface rendering the defined surfaces of the surface image in to a 2D representation of the 3D medical image data. A plurality of ways are known that allow to define the respective surfaces in the surface image. Preferably, the generating of the one or more surfaces in the surface image comprises generating isosurfaces as surfaces, wherein each point of an isosurface is associated with the same predefined isovalue in the medical image data. The predefined isovalue can for instance be defined by a user based on the respective preferences of the user for the visualization of the medical image data. However, the predefined isovalue can also be a standard value for a specific type of medical image, for instance, a standard value for a respective medical imaging procedure to visualize a predetermined anatomical structure. Moreover, the isosurfaces can also be generated based on more than one predefined isovalue, wherein in this case isosurfaces are generated for each isovalue independently and visualized differently, for instance, utilizing different colors. The generating of the isosurface based on an isovalue can be performed, for example, by searching for values equating the isovalue in the medical image data and defining the points of the medical image data to which the respective values belong as part of the surface. Thus the surface image defines all points belonging to the isosurfaces. The surface image can then be visualized by generating a three-dimensional mesh based on the found values in the medical image data that can then be rendered to a 2D representation of the three-dimensional distribution of the values equated to the isovalues in the image data. Additionally, smoothing and/or filtering algorithms can be utilized to smooth the surfaces and remove noise and/or strong outliers that might disturb the respective isosurfaces. Preferably, for generating an isosurface, a marching cube algorithm is utilized based on the predetermined isovalue. A marching cube algorithm can be utilized, in particular, to generate a 3D mesh, e.g., triangles that represents a single isovalue in an isofield or scalar field. For example, for any of a predetermined number of image data values, e.g. eight, of the image data that are neighboring each other and form a cube, the cube is generated with those image data values as corners. Then, for each edge of the cube, see it is determined if image data values on respective two corners of the edge cross the desired isovalue. For instance, if corner A is lower than the desired isovalue and corner B is higher, or vice versa, the position on the edge of the isovalue can be calculated. If A and B are both higher, or, A and B are both lower, the edge can be ignored since the isovalue is not on this edge. Then the marching cube algorithm usually uses a lookup table to create a number of triangles within the cube, for example, based on the corner values, to create a 3D mesh as isosurface. An advantage is that once the marching cube algorithm is performed as a preprocessing step, the resulting 3D mesh can be rendered easily on a GPU taking full advantage of high speed real-time rendering, lighting calculations, shaders etc. provided by the GPU. Moreover, if the isovalue does not change between different views, there is no need to recalculate the 3D mesh such that the algorithm is computational inexpensive. Alternatively, also a raymarching algorithm can be utilized to find the isovalues in the image data.

Additionally or alternatively, at least one of the one or more surfaces in the surface image is generated as cutting surface provided at a predetermined position and angle in the medical image volume through the region of interest. The predetermined position and angle of the cutting surface in the medical image volume can be determined by a user, for instance, by a respective input. However, the cutting surface position and angle can also be predetermined, for instance, can be a standard cutting surface for a specific region of interest. A cutting surface can be generated by determining the points in the image data that belong to the plane with the respective position and angle in the 3D volume representation of the image data. Preferably, the generating of the one or more surfaces in the surface image comprises first generating one or more isosurfaces as surfaces, as explained above, and then combining the one or more isosurfaces with cutting surfaces through the volume of interest. In this case, the cutting surface can either be visualized, i.e. rendered, as visible surface or the cutting plane can be utilized to cut through the one or more isosurfaces, wherein in this case the cutting plane itself is not visible in the visualization but utilized to remove all parts of the one or more isosurfaces on one side of the cutting plane. In this case, for example, first all points belonging to an isosurface as described above are determined and then from these points the points lying on the one or more cutting surface are defined as part of the cutting surface. For example, the generating of isosurfaces for a head of a human being might lead to mainly visualizing the outside of the head of the human being, wherein then a cutting surface can be defined through the head of the human being allowing to additionally visualize isosurfaces within the head of the human being. Generally, it is preferred that image data on at least one side of a cutting surface is ignored during the visualization.

The surface value generation unit is configured for generating a visualization value for a surface point on a surface in the surface image. The visualization value can be generated for all points of the surface but also can be generated only for selected points on the surface. Moreover, a visualization value can be determined also for one or more surface points of more than one surface, for example, for all surfaces or for selected surfaces. For example, if the surface image comprises isosurfaces and cutting surfaces visualization values can be determined only for the isosurfaces or only for the cutting surfaces. Preferably, the surface value generation unit allows a user to select the one or more surface for which one or more visualization values are determined.

Generally, the visualization value is utilized to provide a visualization of information on the surface that is not part of (and/or free from) the respective surface itself, for example, by providing the surface at the surface point with the respective color depending on the visualization value. This allows to provide additional information on the respective surface, wherein the information is not derived from the surface itself. The visualization value is generated based on at least one value of the medical image data associated with a location positioned at a predetermined distance from a location of the surface point and outside the surface itself. Thus, for generating the visualization value, information, i.e. a value of the medical image data, is utilized that is not already presented by the surface itself. Generally, the relation between the visualization value and the at least one value of the medical image data used for generating the visualization value can be any predetermined function. For example, the visualization value can be equated with one value of the medical image data, for instance, a minimum or maximum value of a plurality of values in a predetermined distance from the surface point. However, the visualization value can also have a more complex relation to the at least one value of the medical image data like being generated based on a mean value, weighted mean value, quadratic function, etc. Generally, the relation can be chosen based on a specific application, for instance, based on a specific region of interest, a specific medical imaging procedure, a specific intention for the medical imaging procedure like a searching for a specific illness, and a user might also be allowed to toggle between different relations in order to be provided with different information or to be provided with a preferred view. The at least one value of the medical image data associated with the location positioned at a predetermined distance from a location of a surface point and outside the surface itself can be determined in a plurality of different ways that can depend on the specific application but also on the preferences of the user. For example, in an embodiment, the at least one value of the medical image data utilized for generating the visualization value is located on a ray extending from the respective surface in a predetermined direction and comprises the surface point for which the visualization value is generated. In this case, one or more values for generating the visualization value are located on the ray and thus are located in one direction from the surface allowing for a perception of information in front of or behind the respective surface. In particular, this allows for different perspectives and to project respective information on the surface. Preferably, a direction of a line of sight of a user with respect to the medical image volume is defined and the ray follows the direction of the defined line of sight such that it is parallel to the line of sight. Preferably, the line of sight is defined as the view for which the surface image is rendered into a 2D representation. Following the line of sight allows for a very intuitive providing of information that lies at a predetermined distance in front of and/or behind the surface from the viewing perspective of a user as information on the surface. Changing the line of sight, for instance, by rotating the visualization of the surface in a 2D representation, then automatically allows to change the information projected to the surface. This allows for a very intuitive visualization of the information and their relative position with respect to the surface by changing the line of sight. However, in some embodiments it might also be advantageous that the ray follows a direction with a 90° angle with respect to the surface such that it is normal to the surface. In this way, the information provided on the surface can be standardized and might be better to compare between different cases or different surfaces. In a preferred embodiment, the visualization value is generated utilizing a raymarching algorithm. A raymarching algorithm determines for a respective point in a visualization, for example, a pixel in a 2D image of the image data, a ray from the point through the surface point and utilizes predetermined values of the image data along the ray for determining the visualization value.

As an alternative to utilizing a ray extending from the surface, in an embodiment, the visualization value is generated based on at least one value of the medical image data located inside a predetermined volume surrounding the surface point. In this case, for example, all medical image data values within the volume can be utilized for generating the visualization value, for instance, by averaging utilizing a mean average or by searching for a maximum or minimum value within the predetermined volume surrounding the surface point. In particular, in this way, no important information surrounding the surface is lost.

As described above, the surfaces are preferably cutting surfaces and/or isosurfaces. For the case that the cutting surface is generated as a visible surface, one or more surface values can be generated for the cutting surface and or for the one or more isosurfaces. For the case that the cutting surface is not visible, but used for cutting the one or more isosurfaces, the one or more surface values are generated only for the one or more isosurfaces.

Generally, the surface values can be generated for a cutting surface and/or an isosurface. However, there is a difference for both cases: when using the cutting plane the values from which the visualization value are determined might not comprise the isovalue, whereas when using an isosurface then the values from which the visualization value is determined start from the isovalue. Thus, for algorithms determining the visualization value as relation of the image data values to the isovalue, is preferably utilized for isosurfaces. For example, if the visualization value is indicative of whether values surrounding the isosurface are going up/down with respect to the isovalue, it is preferred the that surface for which the visualization value is determined comprises the isovalue and thus is an isosurface.

The visualization generation unit is then configured for generating a visualization of the medical image volume based on the surface image and the generated visualization value. For example, the visualization generation unit can be configured for rendering the surface image into a 2D representation taking the visualization values into account. In particular, the visualization generation unit can utilize the visualization value to provide the surfaces of the 2D representation of the surface image with the respective information from the visualization value, for instance, utilizing different grey values, colors, symbols, etc. In this way, a visualization of the medical image volume can be generated that not only comprises information about the spatial relation between structures, in particular, anatomical structures visualized by the surface image, but also to provide further information, for instance, on structures or tissues located in front of or behind the surface from the viewing perspective of a user. Thus, by choosing a respective surface, important information that is located slightly in front of or behind the surface will not be lost to the user.

In a further aspect of the invention, a method for generating a visualization of a medical image volume, wherein the method comprises a) providing medical image data acquired from a subject in a medical imaging procedure, wherein the medical image data defines a medical image volume of a region of interest of the subject, b) generating a surface image based on the medical image data defining one or more surfaces in the medical image volume of the region of interest, c) generating a visualization value for a surface point on a surface in the surface image, wherein the visualization value is generated based on at least one value of the medical image data associated with a location positioned at a distance from a location of the surface point and outside the surface itself, and d) generating a visualization of the medical image volume based on the surface image and the generated visualization value.

In a further aspect of the invention, a computer program product for generating a visualization of a medical image volume, wherein the computer program product comprises computer code means for causing an apparatus as described above to carry out the method as described above. It shall be understood that the method, as described above, the apparatus as described above and the computer program product as described above have similar and/or identical preferred embodiments, in particular, as defined in the dependent claims.

It shall be understood that a preferred embodiment of the present invention can also be any combination of the dependent claims or above embodiments with the respective independent claim. These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for visualizing medical image data,
Fig. 2 shows schematically and exemplarily a flowchart of a method for visualizing medical image data, and
Fig. 3 shows schematically and exemplarily a surface image of medical image data.

### DETAILED DESCRIPTION OF EMBODIMENTS

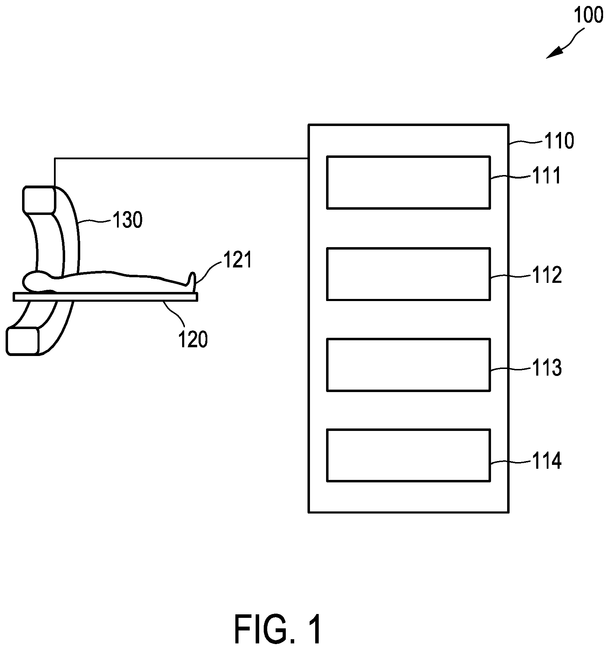

Fig. 1 shows schematically and exemplarily a system for visualizing a medical image volume, wherein the system 100 comprises an apparatus 110 for generating a visualization of a medical image volume. The system 100 comprises an acquisition unit 130 for acquiring medical image data of a subject 121 lying on a patient table 120 in a medical imaging procedure. The medical imaging procedure can be any procedure that allows to generate a 3D scan of at least a region of interest of the subject 121 generating medical image data as result of the scan. For example, the acquisition device 130 can be an MRI, a CT, a 3D ultrasound or a SPECT system. The such acquired medical image data can then be provided to the apparatus 110. However, the medical image data can also be stored on a respective storage not shown in Fig. 1.

The apparatus is configured for generating a visualization of a medical image volume and comprises a medical image data providing unit 111, a surface generation unit 112, a surface value generation unit 113 and a visualization generation unit 114. The medical image data providing unit 111 is configured for providing the medical image data that has been acquired by the acquisition device 130 in the medical imaging procedure. For example, the medical image data providing unit 111 can be configured as an interface that allows to receive the medical image data from the acquisition device 130 for providing the same for the other functional units of the apparatus 110. However, the medical image data providing unit 111 can also be configured to access a respective storage on which the medical image data is already stored and to provide the accessed medical image data to the other functional units of the apparatus 110.

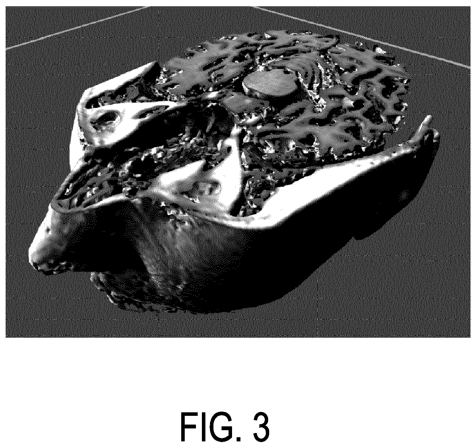

The surface generation unit 112 is configured to generate a surface image based on the medical image data defining one or more surfaces in the medical image volume of the region of interest. Generally, the surfaces generated by the surface generation unit can be any surfaces that define one or more medical image data points as part of the surface. A respective surface image then comprises these respective medical image data points, i.e. the values of the respective medical image data points or another representation of the respective medical image data points that belong to the surface. However, it is preferred that the surface generation unit 112 is configured to generate the surface image as an isosurface image. In this case, an isovalue is predetermined, for instance, by a user or as a standard isovalue and all medical image data points comprising a value equal to the isovalue are determined from the medical image data. The positions of the determined medical image data values corresponding to the isovalue can then be utilized to generate a three-dimensional mesh from the medical image data and for rendering a 2D representation of the three-dimensional surface identified in the medical image data based on the isovalue. An example for such an isosurface image is shown in Fig. 3. The image then shows generally the position of all surfaces that are associated with the isovalue. Thus, all points on an isosurface comprise substantially the same value of the medical image data. However, also other surfaces can be defined, for instance, slices through the 3D volume of the medical image data can be defined by respective positions and angles. In this case, the slice defines which medical image data values belong to the surface. In a preferred embodiment that is shown in Fig. 3, both methods are combined by firstly determining an isosurface image and then defining a slice through the isosurface image such that also isosurfaces that are positioned within other isosurfaces are visible.

The surface volume generation unit 113 is then configured for generating a visualization value for a surface point on the surface of the surface image. Generally, after defining the surfaces in the surface image, the visualization of the surfaces is now defined by the visualization value. Since, as exemplarily shown in Fig. 3, for example isosurfaces only provide information on the position and relation between anatomical structures that comprise the same isovalue in the medical image data, information on structures near the isosurfaces but not comprising the isovalue is completely lost in such a visualization. The visualization value is thus generated for providing a user with at least some information on anatomical structures near a respective surface, for instance, an isosurface. The visualization value is therefore generated based on at least one value of the medical image data that is associated with a location positioned at a predetermined distance from a location of the surface point and outside the surface itself. For example, a quadratic or spherical volume around the surface point for which a visualization value should be determined can be defined with a respective distance, for instance, with a respective radius or with a respective extent and then the maximum or minimum value of the medical image data can be determined as visualization value. However, of course also other relations between the medical image data values surrounding the surface point and the visualization value can be utilized, for instance, depending on the respective application like the respective medical imaging procedure or the respective region of interest. In a preferred embodiment, a ray extending from the surface through the surface point is utilized and one or more medical image data values along this ray are utilized to determine the visualization value. For example, also in this case a minimum or maximum value of the medical image data along the ray can be determined as visualization value, but also a median value, a weighted averaging or any other relation can be utilized. In particular, a user can be provided with a user interface that allows the user to change between different possibilities for generating the visualization value. For example, the user can define the maximum distance of the medical image data value from the surface point that can be utilized for determining the visualization value in this way defining the depth or height, depending on the view of the user, for which information is projected on the surface. Moreover, the user can then also select how the visualization value is generated from the medical image data values within this distance. For example, for some applications it might be useful to simply average all medical image data values within this distance around the surface point. For others, it might be useful to search for the maximum medical image data value within this distance. Generally, the user can also be allowed to toggle between different variations of generating the visualization value in order to compare the results and to provide the user with as much information as possible.

The visualization generation unit 114 is then adapted to generate a visualization of the medical image volume based on the surface image and the generated visualization value. For example, the visualization generation unit 114 can be configured for rendering a 2D representation of the surface image comprising a visualization of the visualization value on the respective surfaces. For example, the visualization value can be visualized for the respective associated surface point with respective colors, grey scales, symbols, etc. This allows to provide the user not only with information on the surfaces themselves, but also on what lies beneath or above the respective surface to a certain extent. Accordingly, it becomes less likely that a user, for instance, a surgeon, misses important information like an important anatomical structure due to a slight misplacement of the isovalue or by utilizing image slices that have been selected too far away from each other to see a specific part of the anatomy, in particular, if this respective part is unexpected.

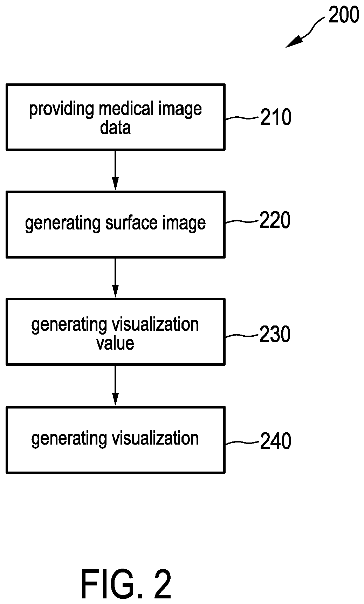

Fig. 2 shows schematically and exemplarily a method 200 for generating a visualization of a medical image volume. In a first step 210, the method 200 comprises providing medical image data acquired from a subject on a medical imaging procedure. In a next step 220, a surface image is generated based on the medical image data defining one or more surfaces in the medical image volume of the region of interest. Then, in step 230, a visualization value is generated for a surface point on a surface in the surface image. The visualization value is generated based on at least one value of the medical image data associated with a location positioned at a distance from a location of the surface point and outside the surface itself. In a last step 240, a visualization of the medical image volume is then generated based on the surface image and the generated visualization value. Generally, the method 200 as described above can be performed by the apparatus 110 and in accordance with the principles described with respect to the apparatus 110.

In the following, some preferred embodiments and examples will be described in more detail. Generally, scans like MRI, CT, or PET produce data in form of a scalar field of values representing the density of matter measured in a 3D volume, e.g., a cube, cut-out part of the body represented as medical image data. Visualization methods usually applied are slicing the volume to a single 2D image cut, or volumetric rendering to a 2D representation of a 3D view, or a combination, e.g., cutting the volumetric rendering to start at a region of interest. However, in all this visualizations information is lost, making it difficult for a user to be provided with all information of the 3D scan in an easy and intuitive manner.

The inventors have found that a visualization method as described above, for example, with respect to Fig. 1 and 2 can be utilized to provide more information to the user in an intuitive manner. For example, in a preferred more detailed embodiment, in a step of the method a scalar field of the 3D scan, i.e. medical image data, can be read from DICOM data and then a marching cubes algorithm can be performed, e.g. on a GPU, to generate a mesh object as surface image of the medical image data. Further, GPU shaders can be utilized for rendering a cutting plane into the surface image, reading back into the original scalar field, i.e. medical image data, at the exact position of the cutting plane. Raymarching from that position into the scalar field, i.e. medical image data, can be utilized to find visualization values that allow to visualize a surface of the surface image as being "thicker" so that more scan information can be used in a single view. Additionally, the visualization can show for example colour coded visualization values that indicated that image data values go up, down or have a specific expert-chosen value in the area around the respective surface point. This allows to give meaning to the matter found behind or in front of the respective surface.

In particular the method can comprise in an embodiment, after first simplifying 3D scan data, i.e. medical image data, by converting it to a mesh, i.e. by determining a surface image, ray marching into the medical image data from a given surface point on that mesh representing the surface. This can then be utilized to visualize the surface "thicker" so that more information about that part of the image data can be provided in a single view.

For example, such an algorithm as described in the embodiments above can be implemented via a software update of a visualization software. In particular, it can be applied on top of an existing visualization software as a new features where the expert might choose raymarching depth and direction, and an algorithm to visualize the values of the raymarching step. Generally, no hardware update or new device is required for this to be used.

In particular, it is preferred that the method is configured for updating software that is configured for generating images and views of a medical 3D scan. Preferably, GPU shaders are utilized at least for a part of rendering processes performed, for example, for generating the visualization. In a first step the DICOM data of the medical scan can be loaded as a scalar field of medical image data to a GPU memory, e.g., a Texture3D. Then, for example, a traditional marching cubes algorithm can be used together with a predetermined isovalue for generating a 3D mesh as part of the surface image. The surface image can then be rendered to a 2D representation using traditional, e.g. GPU-based, rendering methods that can also include cutting planes as shown in Fig. 3. For generating a visualization value of the surface then the GPU shaders can be utilized and can directly render a representation of the visualization value to the respective surface point. In particular, the visualization value can be generated by calculating for a pixel of the surface a 3D position in the 3D scan scalar field, i.e. the medical image data and utilizing a ray marching algorithm for determining a ray from that position on the surface to a user-chosen depth and direction. The such determined ray marched values can then be combined in a plurality of ways into a visualization value that can be rendered as an output color on the surface. Generally, the direction in which to ray is determined gives different results for the visualization value and thus provides different information about structures close to the surface. For example, the determined ray can follow a line of sight of a viewer e.g., by follow a camera direction set in the rendering process. Moreover, the ray can follow a negative normal vector of the surface. Further, the ray can do any of the above however in opposite direction, e.g., looks in front of the surface instead of deeper from the view of the user. Also the ray can be determined in both direction to use data from in front and behind the surface. Moreover, instead of determining a ray also different volumes like a (hemi-)sphere or cube surrounding the surface point can be utilized for determining the visualization value, for example, by sampling the volume at various points be it random or at predefined deltas. Further, when the surface is determined based on a predetermined isovalue, the visualization value can also be determined based on this value. For example, the visualization value can be indicative of whether values of the medical image data go up or down, e.g. on average, with respect to the isovalue along a ray direction. A visualization of the visualization value can then be Boolean colored, for example, green and red or a gradient showing by what amount the medical image data values go up or down. Further, the visualization value can be indicative if a specific value or range around a predetermined value is present along the ray or in the volume, e.g. in the Hounsfield scale. Also a predetermined lookup table can be utilized to determine the visualization value based on a combination of the values along the ray of in the volume. Further, the visualization value can be determined by adding or averaging the values along the ray or in the value. Moreover, a specific visualization value can cause that only this value us rendered such that the rest of the surface image is rendered transparent. Furthermore, the values along the ray or in the volume can also be provided with a weight when determining the visualization value, wherein the weight can be applied based on the distance of the value from the respective surface point, e.g., values closer to the surface get more weight in the visualization value than values farther away.

The invention as described in the examples and embodiments above has the advantage that by compressing more information of a 3D scan into a single view of the scan, diagnostics can be improved, in particular, since medical anomalies are more easily detected. Further, the method can also be utilized for viewing a scan in virtual reality or mixed reality setup allowing a freedom in motion and enabling a more natural way to investigate a scan. This allows also prevent missing a part of the scan more wen viewing compared to going through 2D slices of the scan on a 2D monitor. Having more dense information in a single view therefor reduces the risk of missing important information. Further, different algorithms can be utilized for determining the visualization value and visualize this value, e.g., utilizing different colors, determination possibilities as described above, etc. Thus, the scan can be presented in many ways to the expert again potentially increasing detection of an medical anomaly in the patient's body.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of the medical image data, the generating of the surface image, the determining of the visualization value, etc. performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention refers to an apparatus for generating a visualization of a medical image volume, e.g. MRI, CT, PET. A providing unit provides image data defining an image volume of a region of interest. A generation unit generates a surface image based on the image data defining one or more surfaces in the image volume. A generation unit generates a visualization value for a surface point on a surface in the surface image, wherein the visualization value is generated based on at least one value of the image data associated with a location positioned at a distance from a location of the surface point and outside the surface itself. A generation unit generates a visualization of the image volume based on the surface image and the generated visualization value. This allows to increase the density of the visualized information to a user in one image.

## Claims

1. An apparatus for generating a visualization of a medical image volume, wherein the apparatus (110) comprises:
- a medical image data providing unit (111) for providing medical image data acquired from a subject in a medical imaging procedure, wherein the medical image data defines a medical image volume of a region of interest of the subject,
- a surface generation unit (112) for generating a surface image based on the medical image data defining one or more surfaces in the medical image volume of the region of interest,
- a surface value generation unit (113) for generating a visualization value for a surface point on a surface in the surface image, wherein the visualization value is generated based on at least one value of the medical image data associated with a location positioned at a distance from a location of the surface point and outside the surface itself, and
- a visualization generation unit (114) for generating a visualization of the medical image volume based on the surface image and the generated visualization value.

2. The apparatus according to claim 1, wherein the generating of the one or more surfaces in the surface image comprises generating one or more isosurfaces as surfaces, wherein each point of an isosurface is associated with the same predefined isovalue in the medical image data.

3. The apparatus according to claim 2, wherein for generating an isosurface, a marching cube algorithm is utilized based on the predetermined isovalue.

4. The apparatus according to any of the preceding claims, wherein at least one of the one or more surfaces in the surface image is generated as cutting surface provided at a predetermined position and angle in the medical image volume through the region of interest.

5. The apparatus according to any of the preceding claims, wherein the at least one value of the medical image data utilized for generating the visualization value is located on a ray extending from the respective surface in a predetermined direction and comprises the surface point for which the visualization value is generated.

6. The apparatus according to claim 5, wherein a direction of a line of sight of a user with respect to the medical image volume is defined and the ray follows the direction of the defined line of sight such that it is parallel to the line of sight.

7. The apparatus according to claim 5, wherein the ray follows a direction with a 90° angle with respect to the surface such that it is normal to the surface.

8. The apparatus according to any of claims 5 to 7, wherein the visualization value is generated utilizing a ray marching algorithm.

9. The apparatus to any of claims 1 to 4, wherein the visualization value is generated based on at least one value of the medical image data located inside a predetermined volume surrounding the surface point.

10. A method for generating a visualization of a medical image volume, wherein the method (200) comprises:
- providing (210) medical image data acquired from a subject in a medical imaging procedure, wherein the medical image data defines a medical image volume of a region of interest of the subject,
- generating (220) a surface image based on the medical image data defining one or more surfaces in the medical image volume of the region of interest,
- generating (230) a visualization value for a surface point on a surface in the surface image, wherein the visualization value is generated based on at least one value of the medical image data associated with a location positioned at a distance from a location of the surface point and outside the surface itself, and
- generating (240) a visualization of the medical image volume based on the surface image and the generated visualization value.

11. A computer program product for generating a visualization of a medical image volume, wherein the computer program product comprises computer code means for causing an apparatus according to any of claims 1 to 9 to carry out the method according to claim 10.
